# EUROPEAN PATENT APPLICATION

(11) **EP 4 516 228 A1**
(43) Date of publication of application: **05.03.2025**
(21) Application number: 23194832.4
(22) Date of filing: 01.09.2023
(51) Int. Cl.: A61B 5/369, A61B 5/374, A61B 5/398, A61B 5/00, G16H 50/20

(54) **SYSTEM AND METHOD FOR ESTIMATING SLEEP STAGE**

(71) Applicant: Koninklijke Philips N.V., 5656 AG Eindhoven (NL)
(72) Inventor: VAN GORP, Hans, Eindhoven (NL); DOS SANTOS DA FONSECA, Pedro Miguel Ferreira, Eindhoven (NL); VAN GILST, Merel Marietje, 5656AG Eindhoven (NL); OVEREEM, Sebastiaan, Eindhoven (NL); VAN SLOUN, Ruud Johannes Gerardus, Eindhoven (NL)
(74) Representative: Philips Intellectual Property & Standards

(57) **Abstract**

A mechanism for estimating sleep stages of an subject's sleep session using a machine-learning algorithm. An EOG signal, produced during the sleep session, is obtained. For each of a plurality of samples, a subset of one or more other samples is obtained. The subset is identified by determining temporal dependencies between the sample and the other sample(s) using temporal information trained into the machine-learning algorithm. Each sample is processed using its corresponding subset of one or more other samples to produce an estimate sleep stage for the sample. This produces a plurality of sleep stages for the sleep session of the subject.

## Description

### FIELD OF THE INVENTION

The invention relates to the field of sleep stage scoring.

### BACKGROUND OF THE INVENTION

Sleep stage scoring is used in the diagnosis of many sleep disorders. Sleep stage scoring is conventionally performed by conducting a polysomnography (PSG) study, in which several physiological parameters are measured over a night of sleep. The clinical gold standard for sleep stage scoring is a sleep stage analysis of a PSG measurement conducted by a human technician.

In a PSG study, electroencephalography (EEG), electrooculography (EOG) and electromyography (EMG) measurements are recorded, among others. This requires a subject undergoing a PSG study to sleep while connected to a large number of wires, which can cause discomfort to the subject. Further, performing a full PSG is costly and labor-intensive, and analyzing the PSG to perform sleep stage scoring requires clinical expertise.

There is therefore a need for improved sleep stage scoring techniques.

### SUMMARY OF THE INVENTION

The invention is defined by the claims.

According to examples in accordance with an aspect of the invention, there is provided a processing system for estimating sleep stages of a subject during a sleep session. The processing system is configured to use a machine-learning algorithm configured to: receive an electrooculography, EOG, signal that is responsive to a cornea-retinal standing potential between a front and a back of the eye of the subject during the sleep session; and for each target sample of a plurality of target samples of the EOG signal: identify, as an other sample subset, a subset of one or more other samples in the EOG signal using temporal information to identify temporal dependencies between samples of the EOG signal, wherein the temporal information is defined by a training of the machine-learning algorithm; and estimate a sleep stage associated with the target sample by processing the sample and the other sample subset.

The proposed approach provides a mechanism for accurate estimation of sleep stage for different samples of an EOG signal, e.g., based on a EOG signal having a single channel or an EOG signal having a plurality of channels. This significantly decreases the number of wires to which a subject must be connected in order to perform sleep stage scoring, making sleep stage scoring more comfortable for the subject while providing an estimation of sleep stage that is accurate enough to be clinically useful.

The mechanism proposes using a machine-learning algorithm to identify, for a target sample, one or more other samples that have a temporal dependency with the target sample. The machine-learning algorithm is thereby trained to identify temporal dependencies between samples of the EOG signal. In particular, temporal information is used by the machine-learning algorithm (e.g., forms trained values or coefficients of the machine-learning algorithm) to identify the one or more other samples. The target sample and the identified other samples (labelled an "other sample subset") are further processed by the machine-learning algorithm to identify or estimate a sleep stage for the sample of the EOG signal.

Conceptually, the machine-learning algorithm thereby comprises at least an other sample subset identifying portion and a sleep stage determining portion. The other sample subset identifying portion identifies the other sample subset associated with the target sample. The sleep stage determining portion processes the target sample and the other sample subset to predict the sleep stage for the target sample. The two portions may have been trained simultaneously. Of course, the machine-learning algorithm may comprise one or more other portions, e.g., a pre-processing portion to characterize or extract features from the sample(s) of the EOG signal.

Thus, in the context of the present disclosure the machine-learning algorithm has been trained to identify temporal dependencies, and is therefore capable of taking into account a sleep stage from a sample at the start of the EOG signal when estimating sleep stage for a sample later in the EOG signal (e.g. after several hours of sleep).

The inventors have recognized that, in addition to sleep stage transitions tending to occur between particular sleep stages, sleep stages across a night of sleep have a structured nature that means that a likelihood of a subject being in a particular sleep stage at a particular time in the night depends on the architecture of the sleep that has already occurred during the sleep session. Thus, improved identification of the sleep stage for each target sample can be achieved.

It has been previously mentioned how an EOG signal may comprise multiple channels. Each channel may, for instance, represent a signal measured by a different electrode of an EOG sensing system, e.g., one for each eye of the subject.

The machine-learning algorithm may be configured to, for each target sample, estimate a sleep stage associated with the target sample responsive to a dependency between the timing of the target sample, within the sleep session, and the timing of each other sample in the other sample subset. It has been identified that the relative temporal position between the target sample and other samples that have a temporal dependency on the target sample are significantly influenced by the sleep stage of the target sample. Thus, estimation of the sleep stage of the target sample can be performed more accurately.

In some examples, the timing of the target sample, within the sleep session, and the timing of the samples in the other sample subset are nonconsecutive. For instance, the target sample is not adjacent in time with the samples of the other sample subset. For example, the timing of the target sample and the timing of the samples in the other sample subset are temporally separated from each other by at least 30 seconds, or at least a minute or a least five minutes or at least one hour.

In some examples, the timing of at least one of the samples in the other sample subset is nonconsecutive with the other samples in the other sample subset. For instance, the other sample subset has at least one sample that is not adjacent in time with other samples in the other sample subset. For example, the timing of the at least one sample in the other sample subset is temporally separated from the other samples in the other sample subset by at least 30 seconds, or at least a minute or a least five minutes or at least one hour.

The machine-learning algorithm may be configured to, for each target sample, estimate a sleep stage associated with the target sample responsive to a timing of the target sample within the sleep session. This approach recognizes that different sleep stages occur at different relative frequencies throughout the night. For example, in healthy sleep, sleep stage N3 is more often observed in the first half of the night, while REM sleep is more often observed at the end. Thus, the timing of the target sample within the sleep session provides useful information for improving the accuracy of identifying the sleep stage associated with the target sample.

The machine-learning algorithm may be further configured to: use the temporal information to generate characterizing information for each sample in the EOG signal, including the plurality of target samples; and for each target sample, identify the other sample subset by identifying, as the other sample subset, one or more other samples sharing matching characterizing information to the target sample.

The machine-learning algorithm may be configured to use the temporal information to generate a key, value and query for each sample in the EOG signal, wherein the key represents the characterizing information.

The machine-learning algorithm may be configured to employ an attention-based mechanism to identify, for each target sample, the other sample dataset. The machine-learning algorithm may be a neural network having a transformer architecture.

The input to the machine-learning algorithm may consist of only the EOG signal. In other words, no other physiological signals are input to the machine-learning algorithm in some examples. This means that the subject is only connected to electrodes for acquiring the EOG signal, improving the comfort of the subject compared with a full PSG. As previously mentioned, the EOG signal may comprise a plurality of channels (e.g., each representing a signal or sub-signal generated for each eye).

The EOG signal (input to the machine-learning algorithm) may include electroencephalography, EEG, information. This information is automatically present in an EOG signal, and is usually filtered out. By not filtering the EOG signal to remove EEG information before processing by the machine-learning algorithm, the EEG information may be used to improve the accuracy of the sleep stage estimation. While EOG information is beneficial for wake and Rapid Eye Movement (REM) sleep classification, the EEG information in the signal may be useful for identifying or classifying non-REM sleep (for instance, N3 sleep is characterized by slow-wave cortical patterns).

The EOG signal may be received by the machine-learning algorithm after the sleep session. This allows the machine-learning algorithm to process the EOG signal for the entire sleep session, meaning that later samples in the EOG signal are available to the machine-learning algorithm at the time of estimating a sleep stage for an earlier sample.

The processing system may be further configured to process the estimated sleep stages to generate a hypnogram for the subject. The hypnogram itself represents the state of the subject over the course of a sleep session, thereby providing valuable information about the condition of the subject for the purposes of sleep analysis and/or assessment and/or the diagnosis of one or more sleep conditions.

There is also proposed a processing system for generating temporal information for use in a machine-learning algorithm for estimating sleep stages of a subject during a sleep session. The processing system is configured to: receive a training EOG signal that is responsive to a cornea-retinal standing potential between a front and a back of an eye of a subject during a sample sleep session; receiving a sleep stages reference input representative of sleep stages during the sample sleep session; and process samples of the training EOG signal and the sleep stages reference input to determine temporal dependencies between the samples of the EOG signal; and generate the temporal information responsive to the determined temporal dependencies.

There is also proposed a computer-implemented method for estimating sleep stages of a subject during a sleep session, the computer implemented method comprising using a machine-learning algorithm configured to: receive an electrooculography, EOG, signal that is responsive to a cornea-retinal standing potential between a front and a back of the eye of the subject during the sleep session; and for each sample of a plurality of samples of the EOG signal: identify, as an other sample subset, a subset of one or more other samples in the EOG signal using temporal information to identify temporal dependencies between samples of the EOG signal, wherein the temporal information is defined by a training of the machine-learning algorithm; and estimate a sleep stage associated with the sample by processing the sample and the other sample subset.

The machine-learning algorithm used in the computer-implemented method may be modified analogously to any herein described machine-learning algorithm used by a processing system.

The machine-learning algorithm may be configured to, for each sample, estimate a sleep stage associated with the sample responsive to a dependency between the timing of the sample, within the sleep session, and the timing of each other sample in the other sample subset.

The computer-implemented method may be adapted to carry out the functions of any herein proposed processing system, and vice versa.

There is also proposed a computer program product comprising computer program code means which, when executed on a computing device having a processing system, cause the processing system to perform all of the steps of any herein disclosed method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a workflow in which embodiments can be employed;
Fig. 2 is a flowchart illustrating a proposed method; and
Fig. 3 is a flowchart illustrating another proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for estimating sleep stages of a subject's sleep session using a machine-learning algorithm. An EOG signal, produced during the sleep session, is obtained. For each of a plurality of samples, a subset of one or more other samples is obtained. The subset is identified by determining temporal dependencies between the sample and the other sample(s) using temporal information trained into the machine-learning algorithm. Each sample is processed using its corresponding subset of one or more other samples to produce an estimate sleep stage for the sample. This produces a plurality of sleep stages for the sleep session of the subject.

The present invention recognizes that a significant improvement to the detection or determination of a sleep stage associated with a target sample of an EOG signal can be achieved by identifying temporal dependencies with other samples of the EOG signal. In particular, other samples that have a temporal dependency with the target sample have been identified as having a significant influence on the likelihood as to which sleep stage is associated with the target sample.

Embodiments can be employed in any environment in which the estimation of sleep stages of a sleep session is desired, e.g., for improved understanding of the characteristics of a subject during the sleep session. This information can, for instance, be useful for assessing the condition of the subject and/or making a diagnosis of the subject (e.g., to identify interrupted or shallow sleep, such as that caused by sleep apnea).

The term sleep stage is a well-established term in the field of sleep analysis. Sleep can be typically be classified into at least five stages, including: "awake"; "N1"; "N2"; "N3" and "REM". Each stage may be associated with a different categorical value.

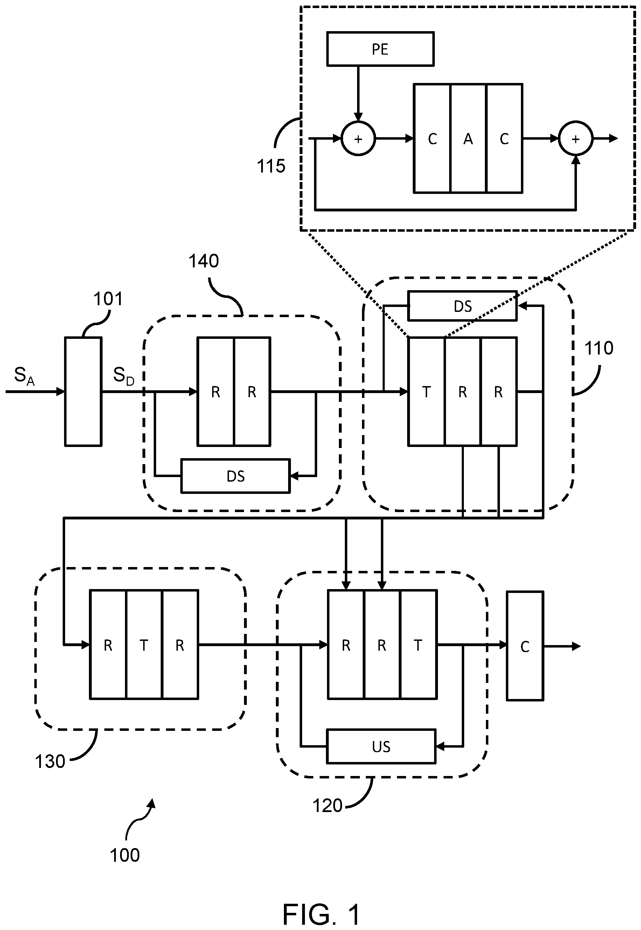

Fig. 1 illustrates an example workflow for obtaining and processing an EOG signal according to a proposed approach, for improved contextual understanding.

In this approach, a (single-lead) analog EOG signal S_{A} obtained during a sleep session is measured and converted to a suitable digital input format by an analog-to-digital converter 101 (ADC), thereby producing a (digital) EOG signal S_{D}. Conversion from the analog domain to a digital domain typically comprises performing sampling and quantization of an analogue signal, here: the analog EOG signal. Approaches for performing analog-to-digital conversion are well known in the art, and are not described in detail for the sake of conciseness.

In the context of the present disclosure, an EOG signal is an electrooculography signal that is responsive to a cornea-retinal standing potential between a front and a back of the eye of the subject during the sleep session.

Further relevant pre-processing steps can also be performed. Such pre-processing steps can be performed in the analog domain and/or the digital domain. Suitable examples of pre-processing processes include signal scaling, signal quality validation and so on.

A machine-learning algorithm 100 is used to process the digital EOG signal S_{D} (hereafter: EOG signal) in order to predict a sleep stage associated with each of a plurality of samples of the EOG signal. It will be clear that the EOG signal is formed from a time-series of values, each value being a sample of the analog EOG signal. A sleep stage is predicted for a plurality of the values/samples (e.g., all of the samples or only some of the samples), which are labelled the target samples.

In some examples, the predicted sleep stages are used to produce a hypnogram of the sleep session. As is well known, a hypnogram is a graph that represents the stages of sleep as a function of time. As each target sample will be associated with a particular point in time, the generation of a hypnogram using the sleep stages for a plurality of samples can be trivially performed.

In determining the sleep stage associated with each target sample, the machine-learning algorithm is configured to effectively identify one or more other samples that have a temporal relationship or dependency with the target sample (for which a sleep stage is to be identified). In particular, the machine-learning algorithm identifies temporal dependencies between the target sample and one or more other samples of the EOG signal. These one or more other samples form an other sample subset, which is processed alongside the target sample to predict the sleep stage associated with the target sample.

Thus, rather than processing each target sample independently or (only) alongside other samples having a fixed temporal relationship with the target sample (e.g., a window of samples centered around the target sample), the proposed approach independently identifies other samples that have a temporal relationship with the target sample. Thus, the collection of samples subsequently processed (to identify a sleep stage for one of the samples) does not have a fixed temporal relationship before being identified by the machine-learning algorithm.

In the proposed approach, the identified other samples do not need to be consecutive or temporally adjacent to any of the other samples and/or the target sample. In particular, each other sample may be temporally distanced from any other sample and/or the target sample.

In preferred examples, the timing of the target sample, within the sleep session, and the timing of the samples in the other sample subset are nonconsecutive. Thus, in some examples, the target sample is not adjacent in time with the samples of the other sample subset. For example, the timing of the target sample and the timing of the samples in the other sample subset are temporally separated from each other by at least 30 seconds, or at least a minute or a least five minutes or at least one hour.

In some examples, there may be at least one further sample (not included in the other sample subset) that is temporally located between the target sample and at least one of the other samples in the other sample subset. In such examples, this further sample is not processed by the machine-learning method in determining the sleep stage for the target sample.

In some examples, the timing of at least one of the samples in the other sample subset is nonconsecutive with the other samples in the other sample subset. In this example, the other sample subset has at least one sample that is not adjacent in time with other samples in the other sample subset. For example, the timing of the at least one sample in the other sample subset is temporally separated from the other samples in the other sample subset by at least 30 seconds, or at least a minute or a least five minutes or at least one hour.

The identifying of the other sample subset, for any given target sample, is performed using temporal information defined by a training of the machine-learning algorithm. The temporal information may, for instance, represent values, coefficients, biases, activation values and/or weights of the machine-learning algorithm that are used to identify the other sample subset. Thus, the temporal information may represent one or more intrinsic values of the machine-learning algorithm that are trained (e.g., modified in a training procedure) to facilitate the identification of an other sample subset of other samples having a temporal dependency with a target sample (for which a sleep stage is to be estimated).

One approach for facilitating the detection of an other sample subset having temporal dependencies with a target sample for which a sleep stage is to be estimated makes use of a machine-learning network having an attention-based mechanism, e.g., one that employs transformers.

The operation, configuration and function of transformers in the context of deep learning or machine-learning algorithms are established in the art. A transformer contains two elements that make it especially suitable for use in a sleep stage estimation technique.

Firstly, the addition of a positional encoding scheme ensures that the machine-learning algorithm is able to effectively see at what time each target sample is located (relative to the overall sleep session). This is useful, since different sleep stages occur at different relative frequencies throughout the night. For example, in healthy sleep N3 is more often observed in the first half of the night, while REM sleep is more often observed at the end.

Moreover, the positional encoding scheme not only gives information about the absolute location of each target sample, but also their relative location with respect to all other samples of the EOG signal.

The self-attention mechanism of a transformer enables it to make long-term associations between different samples. A self-attention mechanism may, for instance, leverage a scheme in which each sample is converted to a Query, Key, and Value. The Query of each sample is used to look or identify matching keys of other samples, and if they match, the value of that sample is propagated to the output to from one of the other samples for the other sample subset.

Together with the positional encoding, the self-attention mechanism of a transformer is able to learn relations between samples at arbitrary time-scales. This overcomes the limited memory and field-of-view constraints of more classical approaches, such as recurrent neural networks.

The present disclosure thereby exploits a machine-learning algorithm that is able to identify other samples having a temporal dependency with the target sample for which the sleep stage is to be estimated to improve the sleep stage estimation of said target sample.

It will be appreciated that once an other sample subset has been identified, the sample and other sample subset are further processed, e.g., in further layers of the machine-learning algorithm, to identify the sleep stage for the target sample.

A machine-learning algorithm is any self-training algorithm that processes input data in order to produce or predict output data. Here, the input data comprises the EOG signal and the output data comprises predicted sleep stages for a plurality of target samples of the EOG signal.

The proposed machine-learning algorithm is configured to identify, for each target sample, an other sample subset comprising one or more other samples having a temporal dependency to/with the target sample. In preferred approaches, the machine-learning algorithm can employ an attention-based mechanism to identify an other sample subset to be associated with each target sample.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

For the present disclosure, if a neural network is used, the neural network may comprise additional layers for performing the other sample subset identification task, e.g., performing the function of the attention identification.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example EOG signals (i.e., training EOG signals). The training output data entries correspond to sleep stages for target samples of the example EOG signals.

The machine-learning algorithm 100 used in the illustrated approach comprises or is in the form of a neural network that processes the EOG signal S_{D} to estimate a sleep stage for each of the plurality of target samples.

The machine-learning algorithm 100 may comprise a U-net architecture. A U-net architecture may be generally formed from an encoding portion 110 and a decoding portion 120. An intermediate portion 130 may process encoded data produced by the encoding portion 110. The encoding portion may perform the function of identifying the sample subset for each target subject.

In practice, the encoding 110 and decoding 120 (portions) may be repeated a plurality of times (represented by the downsampling DS layer or process and upsampling US layer or processing respectively), e.g., 4 times.

By way of example, the encoding portion 110 may comprise one or more transformers 115 that effectively identify the one or more other samples associated with each target sample. The transformer 115 may comprise a self-attention mechanism to identify or establish the samples subset associated with each target sample. The temporal information may form one or more weights, biases, coefficients or other trainable/learnable values of at least the transformer(s) 115.

Examples of suitable transformer architecture(s) include those put forward and/or explained by Vaswani, Ashish, et al. "Attention is all you need." Advances in neural information processing systems 30 (2017); Lee, Juho, et al. "Set transformer: A framework for attention-based permutation-invariant neural networks." International conference on machine learning. PMLR, 2019; Soydaner, Derya. "Attention mechanism in neural networks: where it comes and where it goes." Neural Computing and Applications 34.16 (2022): 13371-13385. Other examples are known to the skilled person.

The machine-learning algorithm 100 may (also) comprise a pre-processing portion 140 that processes the EOG signal before it is processed using the U-net architecture. The pre-processing portion 140 may, for instance, comprise a context encoder for encoding or extracting a context of the (samples of the) EOG signal.

Fig. 1 illustrates one suitable architecture for a machine-learning algorithm. For the purposes of this illustration: the label "R" refers to a residual neural network (ResNet) layer or process; the label "T" refers to a transformer; the label "DS" refers to a downsampling layer or process; the label "US" refers to an upsampling layer or process; the label "C" refers to a convolution layer or process; the label "A" refers to an attention layer (for a transformer) and the label "PE" refers to a positional embedding layer. A positional embedding layer is one that generates information on a relative position of a sample within a sleep session.

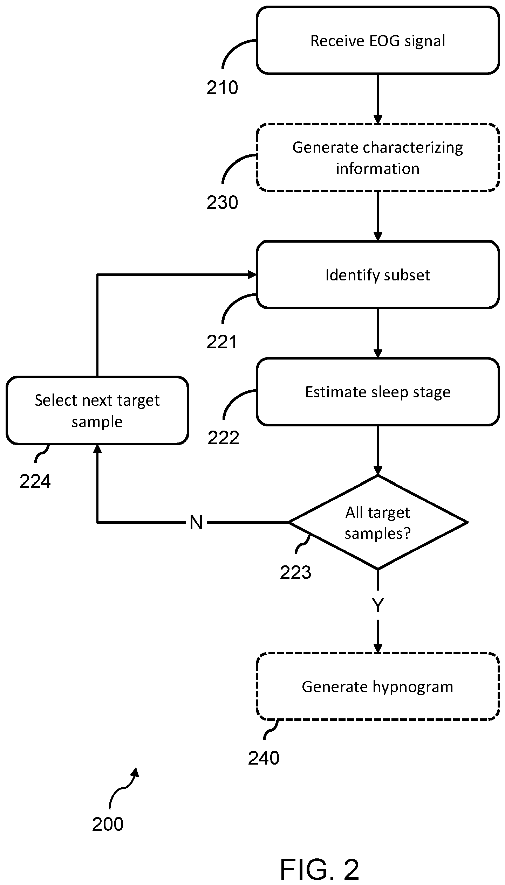

Fig. 2 is a flowchart illustrating steps performed by a machine-learning algorithm 200 for estimating sleep stages of a subject during a sleep session. The machine-learning algorithm may be used or exploited by an appropriately configured processing system, suitable examples of which are later described.

The machine-learning algorithm is configured to receive, in a step 210, an electrooculography, EOG, signal. As previously explained, the EOG signal is responsive to a cornea-retinal standing potential between a front and a back of the eye of the subject during the sleep session.

The machine-learning algorithm is also configured to, for each target sample of a plurality of target samples of the EOG signal, identify (in a step 221) an other sample subset and estimate (in a step 222) a sleep stage associated with the target sample by processing the sample and the other sample subset.

The other sample subset, identified in step 221, is a subset of one or more other samples in the EOG signal. Step 221 is performed by using temporal information to identify temporal dependencies between samples of the EOG signal. The temporal information is defined by a training of the machine-learning algorithm.

In some examples, steps 221 and 222 are repeated for each target sample. For instance, method 200 may, for instance, comprise performing a step 223 of determining, for each iteration of steps 221 and 222, whether all target samples have been processed. Responsive to a negative determination in step 223, the method 200 may perform a step 224 of selecting a next target sample before repeating step 221 for the next target sample. Otherwise, the iterative repetition of steps 221 and 222 may end.

One technique for performing step 221 is to use the temporal information to generate characterizing information for each/every sample (including the plurality of target samples) in the EOG signal. This can be performed in a separate step 230. Step 221 can then be performed by, for each target sample, identifying the other sample subset by identifying, as the other sample subset, one or more other samples sharing matching characterizing information to the target sample.

In this context, the identification of matching characterizing information represents an attempt to identify or establish other pieces of characterizing information that informs the context of the target sample. This may, for instance, include other samples that (in practice) do not exactly match the current sample.

By way of example, step 230 may comprise using the temporal information to generate a key for each sample in the EOG signal. Thus, each sample may have at least a key-value pair, they key characterizing the sample and the value representing the information of the example. The key of a target sample may therefore represent the characterizing information for that target sample. Step 221 may correspondingly comprise identifying keys that match the (key of the) target sample, and including any sample having a matching key in the other sample subset for the target sample.

In some examples, step 230 also generates a query for each sample in the EOG signal. In such approaches, the query of a target sample can be used to search and identify matching keys of samples to be included in the other sample subset, e.g., in step 221.

Suitable mechanisms and techniques for generating a key and/or query for a sample of data are well known in the art, and are not described in detail for the sake of conciseness.

For instance, suitable mechanisms are described by Vaswani, Ashish, et al. "Attention is all you need." Advances in neural information processing systems 30 (2017); or Kitaev, Nikita, Lukasz Kaiser, and Anselm Levskaya. "Reformer: The efficient transformer." arXiv preprint arXiv:2001.04451 (2020). Other suitable examples are well known to the appropriately skilled person in the art.

In particular, step 230 and/or step 221 can be performed by the machine-learning algorithm employing an attention-based mechanism to identify, for each target sample, the other sample dataset. Thus, the machine-learning algorithm may comprise an attention-based algorithmic process, module or portion to identify the other sample dataset for each target sample.

In particular, the machine-learning algorithm may be a neural network, at least a portion of which has a transformer architecture (i.e., the neural network comprises one or more transformers). The transformer architecture can be employed to perform the attention-based mechanism in order to identify the other sample dataset for each target sample, i.e., to carry out steps 230 and 221. Suitable examples of transformers and transformer-based architectures have been previously provided.

Step 222 may be performed by further processing the target sample and the other sample subset using one or more layers or further processing steps of the machine-learning algorithm. These layers are appropriately trained during a training of the machine-learning algorithm.

In step 222, the machine-learning algorithm estimates a sleep stage associated with the target sample responsive to a dependency between the timing of the target sample, within the sleep session, and the timing of each other sample in the other sample subset. Thus, the relative position of the target sample to each other sample in the other sample subset can be used in the determination or calculation of the sleep stage for the target sample.

In step 222, the machine-learning algorithm is configured to, for each target sample, estimate a sleep stage associated with the target sample responsive to a timing of the target sample within the sleep session. Thus, the absolute position of the target sample within the sleep session can be used to improve the estimation of the sleep stage for the target sample.

Of course, it will be appreciated that, in practice steps 221 and 222 may be effectively performed in parallel for all of the target samples of the plurality of target samples. This may, for instance, be performed by the machine-learning algorithm processing the plurality of target samples using a plurality of layers.

In some examples, each sample of the EOG signal is a target sample. Thus, a sleep stage may be predicted for each individual sample of the EOG signal.

The method 200 may further comprise a step 240 of processing the estimated sleep stages to generate a hypnogram for the subject. Approaches for producing a hypnogram from a plurality of estimated sleep stages will be readily apparent to the skilled person. In particular, it will be appreciated that each estimated sleep stage is associated with a sample, whose relative temporal position within the EOG is known. Is it therefore possible to plot the estimated sleep stages against time to produce a hypnogram.

It will be appreciated that the generated hypnogram may be stored (e.g., in a memory or database), be displayed (e.g., at a user interface or the like) and/or undergo further processing (e.g., by a further processing system). The method 200 may be appropriately adapted to include steps for performing such processes.

It has been previously described how an EOG signal is processed by the machine-learning algorithm to generate the predicted sleep stage for each of a plurality of target samples of the EOG signal. As previously mentioned, the electrooculography, EOG, signal is responsive to a cornea-retinal standing potential between a front and a back of the eye of the subject during the sleep session. Thus, an EOG signal can effectively represent a signal component coming from the eyes (an "EOG component")

The EOG signal may be produced by an EOG electrode that is placed sufficiently close to one or both eyes such that the EOG electrode receives neurological signals related to eye movement. An EOG electrode may be an adhesive electrode (that is adhered to a particular location) or a dry electrode (which relies upon direct contact with the skin).

In preferred examples, the EOG signal is a single EOG signal - e.g., produced by a single EOG electrode (e.g., with respect to a reference value) or representing a difference between two potentials measured by two different EOG electrodes (e.g., positioned at either side of an eye). The proposed architecture is advantageous in that it can be readily adapted for use with a single EOG signal.

In some examples, the EOG signal comprises a plurality of channels, e.g., each channel being produced or generated by a different EOG electrode. For instance, the EOG signal may comprise a first channel representing an EOG response at a first eye and a second channel representing an EOG response at a second eye. Other variations will be apparent to the skilled person (e.g., an additional channel representing a differential between the EOG response in the first eye and the second eye). An EOG response is a response to a cornea-retinal standing potential between a front and a back of the eye of the subject during the sleep session.

In general, an EOG signal produced by such an EOG electrode may also be responsive to EEG information. Thus, the (raw) EOG signal produced by an EOG electrode may, in practice, comprise an EOG component (representing a signal coming from the eyes) and an EEG component (representing a signal coming from the cortex) It has traditionally been thought that such EEG information creates noise that should be filtered out before processing, i.e., that any EEG component should be filtered out.

However, in some embodiments of the proposed approach, the EOG signal processed by the machine-learning algorithm has not undergo filtering of any EEG information. In other words, no preprocessing steps to filter out contamination of the EOG signal by EEG are performed.

On the contrary, in some embodiments, the method 200 may actively exploit the fact that such contamination happens. The machine-learning algorithm is effectively able to perform decomposition of the measured signal into the components coming from the eyes and cortex as a result of the identification of the other sample subset for the target sample. This advantageously allows the use of additional information, for predicting a sleep stage, that is historically not been available or usable in sleep stage prediction techniques.

One advantage of the herein proposed approach method, is that it does not require (not per se "automatically" achieve) an explicit decomposition between eye & cortical components. Instead, it leverages commonalities, divergences, and other forms of "cross-information" that present from either source without requiring an explicit separation between the two sources.

The use of both traditional EOG and EEG information is advantageous for the estimation of sleep stage(s), as the signal component coming from the eyes (i.e., the EOG component) is beneficial for Wake and Rapid Eye Movement (REM) sleep classification, while the signal coming from the cortex (i.e., the EEG component) can be used in addition for Non-REM sleep classification: N1, N2, and N3. For example, the accuracy of classifying N3 sleep is significantly improved by the detection of slow-wave patterns in the cortical signal.

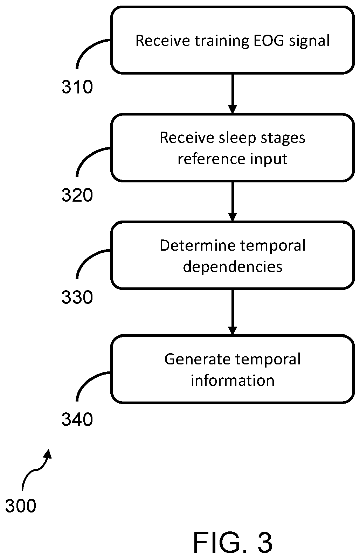

Fig. 3 illustrates a method 300 for generating temporal information for use in a machine-learning algorithm. The method 300 may be performed by a processing system, examples of which are later described.

The method 300 comprises a step 310 of receiving a training EOG signal that is responsive to a cornea-retinal standing potential between a front and a back of an eye of a subject during a sample sleep session.

The method 300 also comprises a step 320 of receiving a sleep stages reference input representative of sleep stages during the sample sleep session.

The method 300 also comprises a step 330 of processing samples of the training EOG signal and the sleep stages reference input to determine temporal dependencies between the samples of the EOG signal.

The method 300 also comprises a step 340 of generating the temporal information responsive to the determined temporal dependencies.

Steps 340 may be performed by performing an iterative process to iteratively update the machine-learning algorithm. The iterative process may comprise modifying one or more values (i.e., learnable values) of the machine-learning algorithm, which together form the temporal information. The iterative process also comprises a step of processing the training EOG signal using the machine-learning algorithm to identify, for each of a plurality of target samples of the training EOG signal, a sample subset. The iterative process may also comprise using a cost function may be used to score the generation of the sample subsets using the determined temporal dependencies between the samples of the EOG signal.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

The machine-learning method used by the processing system may be stored on the processing system itself (e.g., in a memory stored in the processing system) or may be stored and/or executed on an external system. Approaches for storing a machine-learning method and executing or running a machine-learning method using a processing system will be readily apparent to the suitably skilled person.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

In particular, the one or more storage media may store the machine-learning method that is used or executed by the processing system in order to carry out embodiments of the proposed approach. Of course, the one or more storage media may store the temporal information generated by other example embodiments herein proposed.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A processing system for estimating sleep stages of a subject during a sleep session, the processing system being configured to use a machine-learning algorithm configured to:
receive an electrooculography, EOG, signal that is responsive to a cornea-retinal standing potential between a front and a back of the eye of the subject during the sleep session; and
for each target sample of a plurality of target samples of the EOG signal:
identify, as an other sample subset, a subset of one or more other samples in the EOG signal using temporal information to identify temporal dependencies between samples of the EOG signal, wherein the temporal information is defined by a training of the machine-learning algorithm; and
estimate a sleep stage associated with the target sample by processing the sample and the other sample subset.

2. The processing system of claim 1, wherein the machine-learning algorithm is configured to, for each target sample, estimate a sleep stage associated with the target sample responsive to a dependency between the timing of the target sample, within the sleep session, and the timing of each other sample in the other sample subset

3. The processing system of claim 2, wherein the machine-learning algorithm is configured to, for each target sample, estimate a sleep stage associated with the target sample responsive to a timing of the target sample within the sleep session.

4. The processing system of any of claims 1 to 3, wherein the machine-learning algorithm is further configured to:
use the temporal information to generate characterizing information for each sample in the EOG signal, including the plurality of target samples; and
for each target sample, identify the other sample subset by identifying, as the other sample subset, one or more other samples sharing matching characterizing information to the target sample.

5. The processing system of claim 4, wherein the machine-learning algorithm is configured to use the temporal information to generate a key, value and query for each sample in the EOG signal, wherein the key represents the characterizing information.

6. The processing system of any of claims 1 to 5, wherein the machine-learning algorithm employs an attention-based mechanism to identify, for each target sample, the other sample dataset.

7. The processing system of any of claims 1 to 6, wherein the machine-learning algorithm is a neural network having a transformer architecture.

8. The processing system of any of claims 1 to 7, wherein the input to the machine-learning algorithm consists of only the EOG signal.

9. The processing system of any of claims 1 to 8, wherein the EOG signal includes electroencephalography, EEG, information.

10. The processing system of any of claims 1 to 9, wherein the EOG signal is received by the machine-learning algorithm after the sleep session.

11. The processing system of any of claims 1 to 10 further configured to process the estimated sleep stages to generate a hypnogram for the subject.

12. A processing system for generating temporal information for use in a machine-learning algorithm for estimating sleep stages of a subject during a sleep session, the processing system being configured to:
receive a training EOG signal that is responsive to a cornea-retinal standing potential between a front and a back of an eye of a subject during a sample sleep session;
receiving a sleep stages reference input representative of sleep stages during the sample sleep session; and
process samples of the training EOG signal and the sleep stages reference input to determine temporal dependencies between the samples of the EOG signal; and
generate the temporal information responsive to the determined temporal dependencies.

13. A computer-implemented method for estimating sleep stages of a subject during a sleep session, the computer implemented method comprising using a machine-learning algorithm configured to:
receive an electrooculography, EOG, signal that is responsive to a cornea-retinal standing potential between a front and a back of the eye of the subject during the sleep session; and
for each sample of a plurality of samples of the EOG signal:
identify, as an other sample subset, a subset of one or more other samples in the EOG signal using temporal information to identify temporal dependencies between samples of the EOG signal, wherein the temporal information is defined by a training of the machine-learning algorithm; and
estimate a sleep stage associated with the sample by processing the sample and the other sample subset.

14. The computer-implemented method of claim 13, wherein the machine-learning algorithm is configured to, for each sample, estimate a sleep stage associated with the sample responsive to a dependency between the timing of the sample, within the sleep session, and the timing of each other sample in the other sample subset.

15. A computer program product comprising computer program code means which, when executed on a computing device having a processing system, cause the processing system to perform all of the steps of the method according to any of claims 1 to 14.
